# EUROPÄISCHE PATENTSCHRIFT

(11) **EP 0 403 567 B1**
(45) Veröffentlichungstag und Bekanntmachung des Hinweises auf die Patenterteilung: **29.12.1993**
(21) Anmeldenummer: 89904507.4
(22) Anmeldetag: 21.04.1989
(51) Int. Cl.: B65B 9/04, B65B 51/02, A61M 15/00, A61L 9/12

(54) **SPENDER ZUR ABDUNSTUNG VON EINZUATMENDEN WIRKSTOFFEN**
DISPENSER FOR VAPORIZING ACTIVE SUBSTANCES TO BE INHALED
DIFFUSEUR POUR VAPORISER DES PRINCIPES ACTIFS A INHALER

(30) Priorität: 13.07.1988 CH 2661/88; 19.09.1988 CH 3483/88; 21.12.1988 CH 4713/88
(43) Veröffentlichungstag der Anmeldung: 27.12.1990
(73) Patentinhaber: Weick, Heinz Hermann, 1202 Genf (CH)
(72) Erfinder: Weick, Heinz Hermann, 1202 Genf (CH)
(86) Internationale Anmeldenummer: CH8900073
(87) Internationale Veröffentlichungsnummer: WO9000496

(56) Entgegenhaltungen:
- EP-A- 0 179 454
- DE-A- 1 436 767
- DE-A- 3 204 582
- GB-A- 2 184 016
- US-A- 4 285 468

## Beschreibung

Die Erfindung betrifft einen im Bereich des Naseneingangs zu plazierenden Spender zur Abdunstung von einzuatmenden Wirkstoffen medizinischer, paramedizinischer sowie aromatherapeutischer Art, bestehend aus einem den Wirkstoff enthaltenden, als Hohlplättchen ausgebildeten Hohlkörper mit wenigstens einer saugfähigen Abdunstwandung, welche an ihr entlangstreichende Atemluft mit Wirkstoff beaufschlagt, wobei der Hohlkörper eine flache, einen umlaufenden Rand besitzende Schale aus luftdurchlässigem Material aufweist.

Der Bedarf an einfachen Mitteln zur Selbstmedikation mit einzuatmenden Wirkstoffen ist sehr gross, sei es z.B. zur heilwirksamen Behandlung entzündlicher Atemwege oder zur Linderung von Asthma-Beschwerden etc. Zudem kommt der Aromatherapie z.B. gegen Stress, Einschlafbeschwerden usw. ständig grössere Bedeutung zu.

Trotzdem blieb den im Bereich des Naseneingangs zu plazierenden Wirkstoffspendern bisher jeglicher Markterfolg versagt, obwohl seit Generationen verschiedene Konstruktionsvorschläge druckschriftlich bekannt geworden sind. Die Gründe hiefür sind verschiedenster Art. Ihre Raumformen sind ausgeprägt dreidimensional und stellen verhältnismässig grosse Anforderungen an die Herstellung, die sich in einem unangemessen hohen Preis niederschlagen. Bezüglich ihrer Plazierung sind zwei Varianten zu unterscheiden: bei der ersten wird der Spender unter der Nase plaziert und mittels eines den Kopf umspannenden Bandes befestigt, während er bei der zweiten wenigstens teilweise in die Nasenlöcher eingeschoben wird und selbstklemmend in diesen hält. Es bleiben daher entweder ästhetische Anforderungen unerfüllt (grosse dreidimensionale Raumform, Befestigungsband) oder aber physiologische Bedingungen (reizender Kontakt mit der Nasenschleimhaut, Erschwerung des Atmens, Behinderung beim Schnupfen usw.) Mangelhaft ist zudem die Unterbringung des Wirkstoffs in den Spendern. Ist der Spender fabrikmässig mit Wirkstoff gefüllt, muss durch eine gasdichte Einzelverpackung eine Abdunstung während Transport und Lagerung verhindert werden. Die Abdunstintensivität ist nicht dosierbar, obwohl nicht jeder Benutzer eine gleichstarke Wirkstoffbeaufschlagung wünscht oder verträgt. Ein durch Aufträufeln vom Benutzer zu aktivierender Spender dagegen ist unpraktisch. Insbesondere Kindern, kranken und alten Menschen ist eine solche Manipulation kaum zumutbar. Wird dabei der Spender aussenseitig mit Wirkstoff benetzt, so führt das zu unangenehmen Hautreizungen.

Der Erfindung liegt die Aufgabe zugrunde, unter Eliminierung aller den vorbekannten Konstruktionsvorschlägen anhaftender Mängel einen Wirkstoffspender zu entwickeln, der sich in jeder Hinsicht durch eine kompromisslos durchdachte Gestaltung auszeichnet und sich in grossen Stückzahlen automatisch und somit äusserst preiswert herstellen lässt.

Die Erfindung besteht darin, dass das die Schale bildende Material saugfähig ist, dass die Schale durch ein aufgeklebtes Verschlussplättchen abgedeckt ist und mit diesem eine mit den Wirkstoff enthaltenden, durch manuellen Druck zerplatzbaren Mikrokapseln gefüllte Kammer umschliesst, und dass das Verschlussplättchen aussenseitig durch eine mittels einer abziehbaren Folie geschützten, auf der Haut haftenden Klebhaftschicht versehen ist.

Der Wirkstoff des so ausgebildeten Spenders ist von den Mikrokapseln praktisch gasdicht umschlossen, sodass, falls man trotzdem nicht auf eine ebenfalls gasdichte Aussenverpackung verzichtet, durch diese eine doppelte Gassperre erreicht wird. Aktiviert wird der Spender erst unmittelbar vor seiner Verwendung, indem das Hohlplättchen vom Benutzer z.B. zwischen Daumen und Zeigefinger zusammengepresst wird. Dadurch zerplatzen die Mikrokapseln. Der ausfliessende Wirkstoff wird von der saugfähigen, verformten Lage aufgesogen. Da diese ganzflächig abdunstet, ist die wirksame Abdunstfläche maximal gross. Indem wahlweise nur ein mehr oder weniger grosser Teil der Mikrokapseln zerquetscht wird, ist die Abdunstung mehr oder weniger intensiv und eine mehrmalige Aktivierung in beliebigen Zeitabständen möglich. Die Herstellung der Mikrokapseln geschieht kostengünstig in grossen Quantitäten in bekannten Vorrichtungen. Nach Abziehen der Schutzfolie wird der Spender einfachst durch selbsthaftendes Plazieren unter die Nasenscheidewand, den Nasenknorpel oder auch auf die Oberlippe geklebt und lässt sich dort aufgrund seiner kleinflächigen, dünnen Raumform diskret, angenehm und auch während des Schlafens lagesicher tragen. Die an der abdunstenden Fläche entlangstreifende, den Wirkstoff aufnehmende Atemluft wird nicht störend behindert. Ein Kontakt mit der Nasenschleimhaut besteht nicht. Jegliche negative physiologische Nebenwirkung ist somit vermieden. Auch in Bezug auf Materialaufwand und eine automatische Massenfertigung ist die Konstruktion des Spenders kompromisslos durchdacht. Wie aus den anschliessenden Ausführungen zu ersehen, ist die Luftdurchlässigkeit der Schale ein die Fertigung betreffendes wesentliches Konstruktionsmerkmal des Produkts.

Grundsätzlich ist schon bekannt geworden, im Nasen-Mundbereich zu plazierende Wirkstoffspender plättchenförmig flach zu gestalten. Diese sind jedoch verschieden ausgebildet, werden in medizinischen Mundtüchern getragen und dort von der Atemluft durchströmt. Ihre Nachteile bestehen insbesondere im Benutzungsaufwand, der Auffälligkeit ihrer Anwendung und der starken Behinderung der Atemluft. Obwohl einer dieser Wirkstoffspender bereits mikroverkapselten Wirkstoff enthält (japanische Gebrauchsmuster-Veröffentlichung Nr. 54-3595), handelt es sich hier aus vorgenannten Gründen um Produkte verschiedener Art mit den angeführten spezifischen Nachteilen. Zudem lagern beim Wirkstoffspender nach der genannten Druckschrift die Mikrokapseln zunächst in einer Füllung aus absorbierendem textilem Material, welches seinerseits von zwei gelochten, gegeneinander befestigten Schalen ummantelt ist. Der Fabrikationsaufwand ist extrem gross und eine automatische Fertigung kaum denkbar. Von der Textilfüllung werden zudem die schwerer abdunstenden Bestandteile des Wirkstoffes unnötig zurückgehalten, was einer möglichst gleichmässigen Abdunstung der sogenannten Kopf-, Mittel- und Fuss-Note entgegensteht.

Desweiteren zeigt die US-A-4 285 468 einen plättchenförmigen, mit einer Klebhaftschicht versehenen Wirkstoffspender (gemäß dem oberbegriff des Anspruchs 1) zur Plazierung in Räumen wie Toilette, Badezimmer usw. Bei einer spezifischen Ausführung besteht seine Füllung aus kleinen, lose geschüttet eingefüllten Wirkstoffträgern z.B. zylindrischer Form. Sie sind aus einem polymeren, mit Wirkstoff gesättigten Material geformt. Somit ist der Wirkstoff nicht gasdicht umschlossen, sondern im Abdunstkontakt mit der Innenluft des Wirkstoffspenders. Demzufolge ist dieser wenigstens auf seiner Abdunstseite zweischichtig ausgebildet und weist eine gasdichte äussere, vor Gebrauch abziehbare, z.B. aus Aluminium bestehende Folie, sowie eine innere gasdurchlässige Wandung aus polymerem Material auf. Der Material- und Herstellungsaufwand ist dementsprechend gross. Die spezifische Abdunstleistung des gasdurchlässigen polymeren Wandungsmaterials ist bedeutend geringer als diejenige der saugfähigen Schalenwandung entsprechend vorliegender Erfindung. Würde man diesen Wirkstoffspender kleiner dimensionieren und seine hohen Kosten akzeptieren, bliebe er trotzdem für eine Selbstindikation im Sinne des erfindungsgemässen Produkts völlig ungeeignet.

Zu erwähnen ist noch, dass zum Desodorisieren, Parfümieren, sowie zur Abdunstung von Insektiziden dienende, plättchenförmig flache, auf die Kleidung oder z.B. in den Achselhöhlen direkt auf die Haut zu klebende Wirkstoffspender bereits druckschriftlich bekannt geworden sind. Sie haben nichts mit einer Selbstindikation zu tun, sondern mit allgemeiner äusserer Körperpflege, bzw. Insektenabwehr, betreffen also eine andere Produktgattung. Zudem sind Konstruktion sowie Art und Plazierung des Wirkstoffs innerhalb des Hohlplättchens und damit auch ihr Herstellungsverfahren gegenüber dem erfindungsgemässen Produkt verschieden und unausgereift.

Nach weiterer Erfindung besteht das Verfahren zur automatischen Herstellung des Wirkstoffspenders nach Anspruch 1 darin, dass in einer Fabrikationsstrasse zunächst eine Lage saugfähigen, luftdurchlässigen Materials mittels einer Presseinrichtung wenigstens eine schalenförmige Verformung erhält, welche anschliessend durch eine Fülleinrichtung mit Mikrokapseln beschickt und dann mittels einer ersten Kaschiereinrichtung mit einer das Verschlussplättchen bildenden zweiten Lage überklebt wird, wonach ein Klebstoffauftragswerk die zweite Lage aussenseitig mit einer Kelbhaftschicht versieht, die mittels einer zweiten Kaschiereinrichtung mit einer abziehbaren Schutzfolie überdeckt wird, und dass die schalenartige Verformung wenigstens vor Beginn ihrer Beschickung mit durch manuellen Druck zerplatzbaren Mikrokapseln bis zu ihrer Ueberklebung mit der zweiten Lage durch ein gegengleiches Lagerbett einer Trägerplatte mit Saugluft beaufschlagt wird, die das luftdurchlässige Material im Bereich der Verformung durchströmt.

Durch dieses Verfahren wird unter anderem das schwierige Problem des Manipulierens der winzigen, leichten und daher zum Zerstreuen neigenden Mikrokapseln auf einfachste und sicherste Art gelöst. Durch die Beaufschlagung der Trägerplattenlagerbetten mit die saugfähige Lage im Bereich der Verformung durchströmender Saugluft werden die von oben her in loser Schüttung einfliessenden Mikrokapseln ohne jegliche Streuneigung in die Verformung eingesogen und derart sicher in dieser gehalten, dass die Trägerplatte anschliessend mit hoher Beschleunigung zur nächsten Station der Fabrikationsstrasse verfahrbar ist. Zudem bewirkt die genannte Saugluftbeaufschlagung eine gute, auf Zug beanspruchbare Lagesicherung der verformten Lage in der Trägerplatte, wodurch die Materialbahn beim Verfahren der Trägerplatten automatisch nachgezogen wird.

Die erfindungsgemässe Fabrikationsstrasse zur automatischen Durchführung des Verfahrens nach Anspruch 10 ist gekennzeichnet durch eine Prägepresse zur schalenartigen Verformung einer Lage saugfähigen, luftdurchlässigen Materials, der eine Fülleinrichtung zur Beschickung der schalenartigen Verformung mit durch manuellen Druck zerplatzbaren Mikrokapseln folgt, an die sich eine erste Kaschiereinrichtung zur Ueberklebung der gefüllten Verformung mit einer Abdecklage anschliesst, welcher ein Klebstoffauftragswerk zur Aufbringung einer Klebhaftschicht aussenseitig auf die Abdecklage, eine zweite Kaschiereinrichtung zur Abdeckung der Klebhaftschicht mit einer silikonartigen abziehbaren Schutzfolie, sowie eine Einrichtung zur automatischen Entnahme des fertigen Wirkstoffspenders folgen, weiterhin durch in Materialflussrichtung etwa von der Prägepresse bis zur Entnahmeeinrichtung verfahrbare, die schalenartige Verformung der verformten Lage in gegengleichen Lagerbetten aufnehmende Trägerplatten, welche Lagerbetten luftdurchlässig ausgebildet und von ihrer Unterseite her mit einer Einrichtung zur Saugluftbeaufschlagung verbunden sind.

Weitere, den Wirkstoffspender, das Verfahren zu seiner Herstellung sowie die Fabrikationsstrasse zur Durchführung des Verfahrens betreffende Erfindungsmerkmale und aus diesen resultierende weitere Vorteile gehen aus der Figurenbeschreibung hervor.

In der Zeichnung sind Ausführungsbeispiele der Erfindung dargestellt. Es zeigen:
- Figur 1: eine Draufsicht eines erfindungsgemässen Wirkstoffspenders,
- Figur 2: einen Seitenriss nach Fig. 1,
- Figur 3: einen Schnitt I-I nach Fig. 1,
- Figur 4: das Gesichtsprofil einer einen Wirkstoffspender nach den Fig. 1 - 3 tragenden Person,
- Figur 5: einen Seitenriss einer schematisch dargestellten Fabrikationsstrasse zur automatischen Herstellung des erfindungsgemässen Wirkstoffspenders,
- Figur 6: eine Draufsicht nach Fig. 5,
- Fig.7a - 7c: das Prinzip der Beschickung der verformten Lage mit Mikrokapseln,
- Figur 8: einen vergrösserten Schnitt eines Konstruktionsdetails der Beschickungseinrichtung nach den Fig. 7a - 7c,
- Figur 9: das Prinzip der Entnahme der fertigen, ausgestanzten Wirkstoffspender in Schnittdarstellung und
- Figur 10: die Draufsicht eines eine Mehrzahl von Wirkstoffspendern enthaltenden Montageblocks.

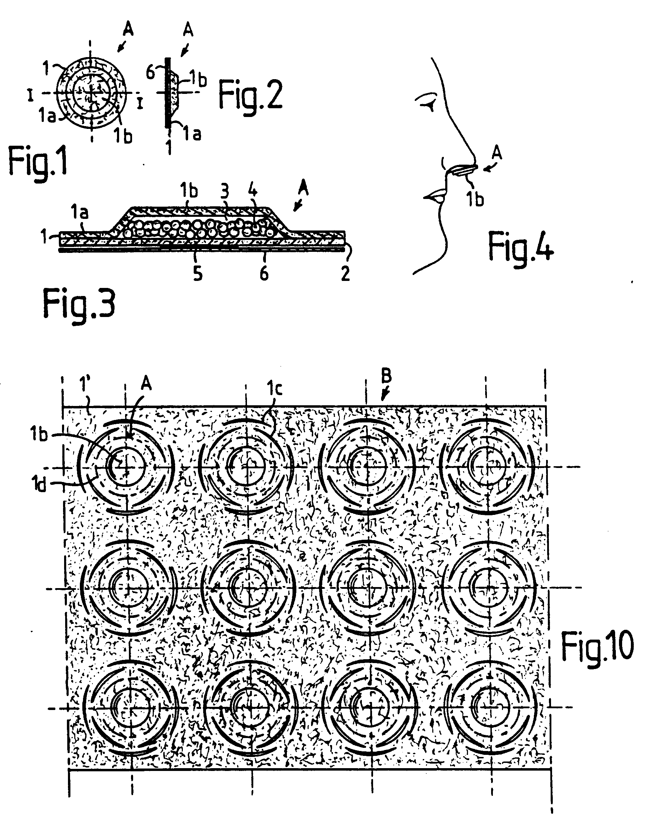

Der Wirkstoffspender nach den Fig. 1 - 4 besteht zunächst aus einer flachen, von einem umlaufenden Rand la umgebenen Schale 1 aus saugfähigem, luftdurchlässigem Material wie ungeleimtem, wenig gepresstem Papier, Fliess oder dergleichen, welche mittels eines auf den Rand la aufgeklebten Verschlussplättchens 2 z.B. gleichen Materials verschlossen ist. In der dadurch gebildeten geschlossenen Kammer 3 befinden sich lose geschüttet mit medizinischem Wirkstoff gefüllteMikrokapseln 4 mit einem Durchmesser von beispielsweise 500 - 800µ. Aussenseitig ist das Verschlussplättchen 2 in seinem zentralen Bereich mit einer tupferartigen Klebhaftschicht 5 belegt, die ihrerseits von einer abziehbaren silikonisierten Schutzfolie 6 überdeckt ist.

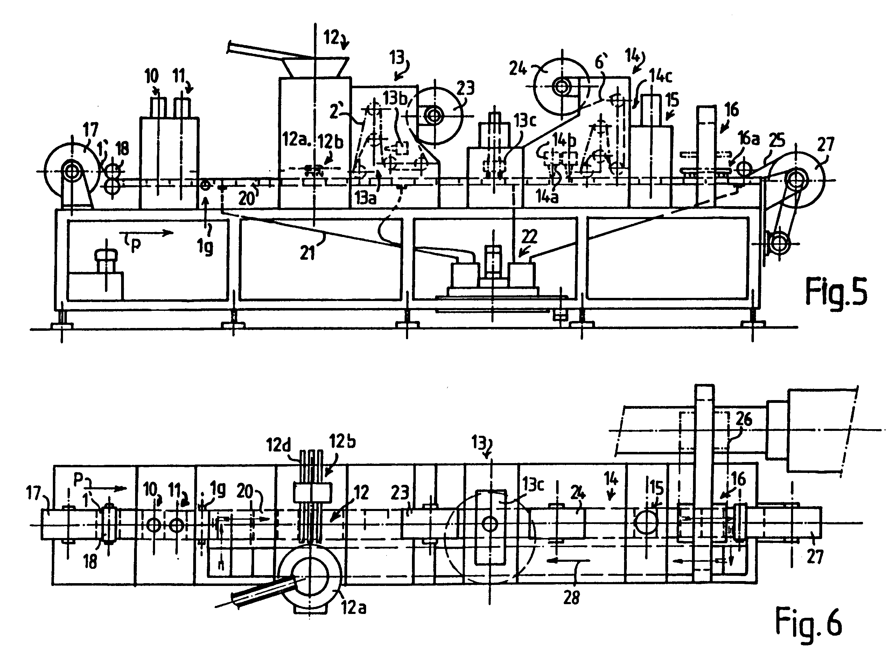

Zur Aktivierung des Wirkstoffspenders wird der Wirkstoff durch einen manuellen Druck auf die Schale 1, d.h. durch Zerquetschen der Mikrokapseln 4 freigesetzt und unmittelbar vom saugfähigen Material der Schale 1 zu seiner Abdunstung nach aussen geleitet. Nachdem der Benutzer anschliessend die Schutzfolie 6 entfernt hat, klebt er sich den Wirkstoffspender entsprechend Fig. 4 unter die Nasenscheidewand, den Nasenknorpel oder auf die Oberlippe. So plaziert streicht die Atemluft aussen an der Schale 1 entlang und nimmt Wirkstoff auf, der somit auf kürzestem Weg in den Körper gelangt. Zum Einsatz kommen Wirkstoffe medizinischer, bzw. paramedizinischer Art, wie z.B. Eukalyptusöl bei entzündlichen Erkrankungen der Atemwege, sowie solche der immer mehr an Bedeutung gewinnenden Aromatherapie.

Die Vorteile des derart kompromisslos konzipierten Wirkstoffspenders sind folgende:
Bis zu seiner Benutzung ist der Wirkstoff gasdicht in den Mikrokapseln 4 eingeschlossen, wodurch eine gasdichte Aussenverpackung verzichtbar ist.

Die Aktivierung des Wirkstoffes geschieht durch einen simplen manuellen Druck auf die Schale 1, wodurch die Mikrokapseln 4 zerplatzen.

Die Schale 1 dunstet ganzflächig ab, d.h. die Abdunstfläche ist maximal gross.

Die Abdunstleistung ist dosierbar, indem in gewünschten Zeitabständen jeweils durch leichterten Druck auf die Schale 1 nur ein Teil der Mikrokapseln 4 zerquetscht wird.

Die Plazierung durch Selbstklebehaftung ist denkbar einfach und bei jeweils nur teilweiser Aktivierung der Mikrokapseln ein und desselben Spenders mehrmals wiederholbar.

Infolge der praktisch zweidimensionalen Ausbildung ist der Spender diskret zu tragen und auch während des Schlafens nicht störend.

Es besteht praktisch weder eine Atemluftbehinderung noch irgendein Kontakt mit der Schleimhaut, d.h. physiologische Nebenwirkungen sind ausgeschaltet.

Die Luftdurchlässigkeit der Schale 1 und die gegenüber dem Durchmesser des Spenders kleinere Klebhaftschicht 5 erlauben eine ausserordentlich preiswerte Massenfertigung (Ansaugbeschickung mit Mikrokapseln 4 - von der Klebhaftschicht 5 ungehindertes Ausstanzen).

Die Schutzfolie 6 lässt sich zwecks Entfernens vom gesamten Umfang des Spenders her leicht greifen.

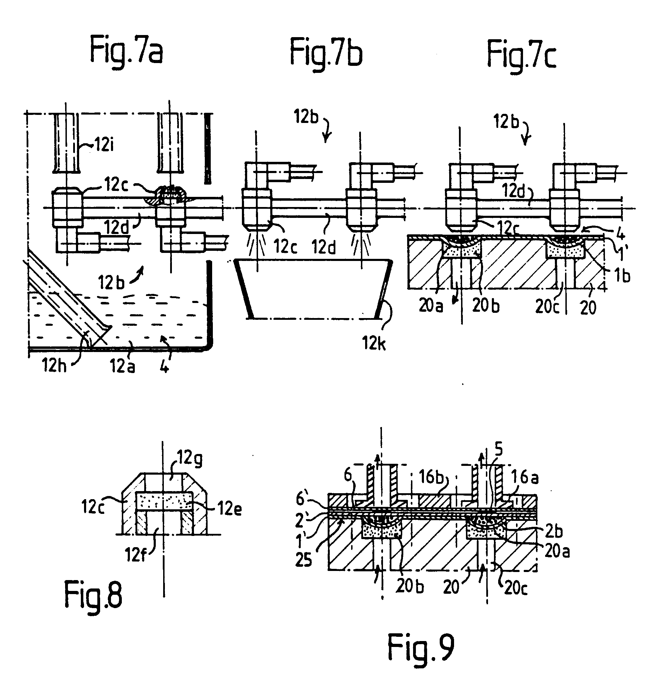

Die in den Figuren 5 und 6 schematisch dargestellte Fabrikationsstrasse zur automatischen Fertigung des Wirkstoffspenders ist taktgesteuert, d.h. der Materialtransport erfolgt schrittweise. Die Flussrichtung des Materials ist durch den Pfeil P angezeigt. Die Figuren 7a - 7c, 8 und 9 zeigen in ausführlicherer Darstellung besonders wichtige Konstruktionsdetails.

In Materialflussrichtung P hintereinander angeordnet weist die Fabrikationsstrasse die folgenden wesentlichen Einrichtungen auf:
Stanzeinrichtung 10 zur Erzeugung von Trennschnitten 1c in der Lage l' saugfähigen, luftdurchlässigen Materials; Prägepresse 11 zur Erzeugung der schalenartigen Verformungen 1b in der genannten Lage; Fülleinrichtung 12 zur Beschickung der schalenartigen Verformungen 1b mit Mikrokapseln 4; erste Kaschiereinrichtung 13 zur Ueberklebung der verformten, mit Mikrokapseln 4 beschickten Lage 1' mittels einer Abdecklage 2'; zweite Kaschiereinrichtung 14 zur tupferartigen Aufbringung einer Klebhaftschicht 5 auf die Abdecklage 2' und Abdeckung der Klebhaftschicht 5 mittels einer abziehbaren Schutzfolie 6'; Stanzeinrichtung 15 zum Ausstanzen der fertigen Wirkstoffspender A sowie eine Einrichtung 16 zu deren automatischen Entnahme.

Die Lage 1' saugfähigen, luftdurchlässigen Materials (z.B. ungeleimtes, ungepresstes Papier) wird von der drehbar gelagerten Materialrolle 17 aus mittels der Umlenkrollen 18 zur Stanzeinrichtung 10 umgeleitet.

Die Fabrikation geschieht blockweise, d.h. jede Operation betrifft gleichzeitig eine Mehrzahl von zu fertigenden, in Längs- und Querreihen als Montageblock B zusammenhängenden, zuletzt aus diesem auszustanzenden Wirkstoffspendern A. Anzahl und gegenseitiger Abstand der Wirkstoffspender A eines Montageblocks B sind durch die Grösse der Verpackungseinheit gegeben. Bei entsprechend grosser Auslegung der Fabrikationsstrasse ist es natürlich möglich, zwecks weiterer Senkung der Herstellungskosten unter gleichzeitiger Kapazitätserhöhung mehrere Montageblöcke, d.h. eine Montageblockgruppe pro Arbeitstakt zu fertigen.

Eine brauchbare Verformung einer montageblockgrossen Lage 1' saugfähigen, luftdurchlässigen, im trockenen Zustand kaum ziehfähigen Papiers ist an sich nur während der Papierherstellung möglich und somit mit grossen Umständen verbunden. Mit der Stanzeinrichtung 10 wird dieses Handicap umgangen. Sie stanzt entsprechend Fig. 10 in einen für einen Montageblock B bzw. eine Montageblockgruppe bemessenen Flächenbereich der entsprechenden Lage 1' im Abstand um jede zu erzeugende schalenartige Verformung 1b, die koaxialen, sich überlappenden Freischnitte 1c, sodass die anschliessend erzeugten Verformungen 1b stegförmig mit dem ganzen verbunden bleiben.

Während der durch die Formpresse 11 erfolgenden Verformung ziehen sich die Freischnitte 1c im erforderlichen Masse auseinander, sodass sich eine (nicht gegebene) Ziehfähigkeit des Materials erübrigt.

Im Bereich der Formpresse 11 befindet sich wenigstens eine quer zur Transportrichtung ausgerichtete, die Lage 1' untergreifende, senkrecht bewegbare Hubrolle 19, mit der die genannte Lage zwecks ihres Weitertransports aus der Matrize des nicht dargestellten Presswerkzeugs herausgehoben wird. Die Hubrolle 19 ist mit nicht sichtbaren Ringnuten versehen, durch die die Verformungen 1b unbehindert hindurchgleiten können. Nach dem Transportschritt und dem Wiederabsenken der Hubrolle 19 wird die verformte Lage 1' von einer verfahrbaren Trägerplatte 20 aufgenommen Eine Mehrzahl solcher Trägerplatten 20 werden, den Materialtransport übernehmend, schrittweise von der Formpresse 11 aus bis zur Entnahmeeinrichtung 16 verfahren, um von dort aus im Bewegungskreislauf auf der parallelen Bahn 28 zum Ausgangspunkt zurückgefahren zu werden. Zur Aufnahme der Verformungen 1b besitzen die Trägerplatten 20 Lagerbetten 20a. Diese sind von unten her über einen luftdurchlässigen Einsatz 20 b z.B. aus Sintermetall und eine Bohrung 20c mittels der Leitungen 21 über ein Karussell 22 bedarfsweise mit Saug- oder Druckluft beaufschlagbar. Beaufschlagt mit Saugluft ist die verformte Lage 1' derart auf der Trägerplatte 20 lagegesichert, dass diese bei ihrer schrittweisen Bewegung die Papierbahn automatisch nachzieht.

Die Fülleinrichtung 12 besitzt einen seitlich der Fertigungsstrasse plazierten Dosierbehälter 12a und ein saugheberartig wirkendes, zwischen diesem und der jeweils das Füllgut übernehmenden Trägerplatte 20 hin und her verfahrbares Dosierorgan 12b. Dieses ist mit Dosierköpfen 12c bestückt, deren Anzahl und gegenseitige Anordnung den Verformungen 1b eines Montageblocks B, bzw. einer Gruppe von Montageblocks B entspricht. Die jeweils in Querrichtung der Fabrikationsstrasse in Reihe angeordneten Dosierköpfe 12c sind auf drehbaren Axen 12d angeordnet. Jeder Dosierkopf 12c besitzt einen einseitig offenen, durch einen luftdurchlässigen, z.B. von einem eingesetzten Sintermetallplättchen 12e gebildeten Boden und die Bohrung 12f her bedarfsweise mit Saug- oder Druckluft beaufschlagten Dosierraum 12g. Zur dosierten Uebernahme von Mikrokapseln 4 befinden sich die Dosierköpfe 12c mit nach oben gerichteten Dosierraumöffnungen im Dosierbehälter 12a (Fig. 7a). Hier werden die Dosierräume durch eine pneumatische Transporteinrichtung 12h, 12i von oben her mit Mikrokapseln 4 beschüttet und in diesen durch deren gleichzeitige Saugluftbeaufschlagung gehalten. Eventuell überfliessendes Schüttgut fällt unten in den Dosierbehälter 12a zurück. Das Dosierorgan 12b wird nun bei einer gleichzeitig erfolgenden 180 Drehbewegung der Axen 12b über die jeweils zu beschickende, mit Saugluft beaufschlagte Trägerplatte 20 gefahren (Fig. 7c). Indem nun die Saugluftbeaufschlagung der Dosierköpfe 12c unterbrochen wird, fallen die Mikrokapseln 4 nicht nur in die Verformungen 1b hinein, sondern werden durch den Luftdurchlässigkeit ohne jede Streuwirkung förmlich in sie hineingesogen und lagegesichert. Da den Mikrokapseln 4 zur Vermeidung gegenseitigen Klebens fabrikmässig etwas pulverartige Substanz beigegeben wird, könnte sich das Sintermetallplättchen 12e aufgrund der Sauglkuft verstopfen. Dem wird dadurch entgegengewirkt, dass die Dosierköpfe 12c auf ihrem Rückweg zum Dosierbehälter 12a über einem Trichter 12k durch einen kurzen Druckluftstoss ausgeblasen werden.

Durch diese Mittel wird die an sich äusserst schwierige Manipulation der nur sehr geringe mechanische Beanspruchung ertragenden und aufgrund ihrer Winzigkeit und geringen Masse sich durch den geringsten Lufthauch zerstreuenden Mikrokapseln hervorragend gelöst. Ihre einzige mechanische Belastung besteht aus gegenseitiger Reibung sowie geringfügiger Wandungsreibung während des pneumatischen Transports und in den Dosierräumen 12g. Die Trägerplatten 20 lassen sich mit grosser Beschleunigung zur nächsten Station verfahren, ohne dass eine Mikrokapsel aus den Verformungen 1b entweicht.

Innerhalb der Kaschiereinrichtung 13 wird die z.B. ebenfalls aus Papier bestehende Abdecklage 2' von der drehbar gelagerten Materialrolle 23 aus durch die Umlenkrollen 13a in die Trägerplattenebene umgelenkt. Mit dem Klebstoffauftragswerk 13b wird die Lage 2' unterseitig mit erhitztem, schnellhärtendem Klebstoff beschichtet. Die gegenseitige Klebverbindung zwischen der Verformten Lage 1' und der Abdecklage 2' geschieht durch den den Klebstoff durch Aufheizung reaktivierender Heizstempel 13c.

Die zweite Kaschierstation 14 enthält zunächst das mit düsenähnlichen Organen 14a bestückte Klebstoffauftragswerk 14b. Mittels der Düsen 14a wird die Abdecklage 2' zentral über einer jeden Verformung 1b mit einem tupferartigen Klebhaftpunkt bzw. strich 5 versehen. Die anschliessend aufzukaschierende silikonisierte Schutzfolie 6' wird von der drehbar gelagerten Materialrolle 24 aus über die Umlenkrollen 14c auf die mit Klebhafttupfern versehene Abdecklage 2' aufgebracht.

Nun erfolgt die Ausstanzung aller Wirkstoffspender A des fertigen Montageblocks B, bzw. der Montageblockgruppe mittels der Stanzvorrichtung 15. Da die Verklebung zwischen der verformten Lage 1' und der Abdecklage 2' mittels eines unmittelbar härtenden, sogenannten "hot-melt" vorgenommen ist, und die auszustanzenden Wirkstoffspender A nur zentral tupferartig mit Klebhaftschicht 5 versehen sind, kommen die Stanzwerkzeuge nicht mit klebender Substanz in Berührung, wodurch sich ein ständiges lästiges Reinigen erübrigt und entsprechende Störungen ausgeschaltet sind. Hinzu kommt noch, dass sich bei so ausgelegter Klebhaftschicht 5 die Schutzfolie 6 vom gesamten Umfang her zwecks Entfernens leicht greifen lässt.

Die als Händler ausgebildete Entnahmevorrichtung 16 ist mit saugluftbeaufschlagbaren Entnahmeplättchen 16a bestückt (Fig. 9), die in Anzahl und gegenseitiger Anordnung den in einem Montageblock B, bzw. einer Montageblockgruppe vorhandenen Wirkstoffspendern A entsprechen. Während die Entnahmeplättchen 16a die Wirkstoffspender A ansaugen und der Niederhalter 16b den Verschnitt 25 des Montageblocks A bzw. der Montageblockgruppe zurückhält, werden die Lagerbetten 20a der entsprechenden Trägerplatte 20 gleichzeitig mit Druckluft beaufschlagt, wordurch die Wirkstoffspender A zusätzlich gegen die Entnahmeplättchen 16a gedrückt werden. Dadurch ist verhindert, dass sich während dieser Operation die Schutzfolie 6 vom Klebhafttupfer 5 lösen kann. Vom Händler 16 werden die Wirkstoffspender nun unmittelbar in die von einem Verpackungsautomaten zugeführte Verpackung 26 umgesetzt. Der als Band zusammenhängende Verschnitt 25 wird zur Rolle 27 aufgewickelt.

Ein weiterer Vorteil der Fabrikationsstrasse ist durch eine spezielle (nicht dargestellte) Ausgestaltung der Füllstation 12 erreichbar. Diese besteht darin, dass der Dosierbehälter 12a mit mehreren, z.B. drei Kompartiments zur Füllung der Dosierräume 12g mit Mikrokapseln 4 versehen ist. Dadurch lassen sich z.B. die Dosierköpfe 12c einer jeden Achse 12d gleichzeitig mit Mikrokapseln verschiedener Wirkstoffe bzw. Aromen beaufschlagen. Eine jede Reihe von Spendern in der Verkaufsverpackung 26 enthält dadurch einen anderen Wirkstoff bzw. einen Wirkstoff mit verschiedenem Aroma. Die Reihen sind auf der Verpackung entsprechend gekennzeichnet. Der Verbraucher hat somit die Möglichkeit, z.B. bei einer Selbstmedikation zur Beruhigung oder zum leichteren Einschlafen den Wirkstoff bzw. das Aroma zu wechseln.

## Patentansprüche

1. Im Bereich des Naseneingangs zu plazierender Spender zur Abdunstung von einzuatmenden Wirkstoffen medizinischer, paramedizinischer sowie aromatherapeutischer Art, bestehend aus einem den Wirkstoff enthaltenden, als Hohlplättchen (A) ausgebildeten Hohlkörper mit wenigstens einer saugfähigen Abdunstwandung, welche an ihr entlangstreichende Atemluft mit Wirkstoff beaufschlagt, wobei der Hohlkörper eine flache, einen umlaufenden Rand (1a) besitzende Schale (1) aus luftdurchlässigem Material aufweist, dadurch gekennzeichnet, dass das die Schale (1) bildende Material saugfähig ist, dass die Schale (1) durch ein aufgeklebtes Verschlussplättchen (2) abgedeckt ist und mit diesem eine mit den Wirkstoff enthaltenden, durch manuellen Druck zerplatzbaren Mikrokapseln (4) gefüllte Kammer (3) umschliesst, und dass das Verschlussplättchen (2) aussenseitig durch eine mittels einer abziehbaren Folie (6) geschützten, auf der Haut haftenden Klebhaftschicht (5) versehen ist.

2. Spender nach Anspruch 1, dadurch gekennzeichnet, dass die Klebhaftschicht (5) gegenüber dem Verschlussplättchen (2) kleinflächiger bemessen ist, derart, dass der gesamte Umfangsbereich des Verschlussplättchens (2) klebhaftschichtfrei ist, und dass die Schutzfolie (6) und das Verschlussplättchen (2) grössen- und deckungsgleich sind.

3. Spender nach Anspruch 2, dadurch gekennzeichnet, dass das Verschlussplättchen (2) mit einer etwa mittig angeordneten, etwa punktförmigen Klebhaftschicht (5) versehen ist.

4. Spender nach einem der Ansprüche 1 - 3, dadurch gekennzeichnet, dass das Hohlplättchen (A) eine kreisrunde Aussenkontur mit einem Durchmesser von etwa 15 - 20 mm aufweist.

5. Spender nach einem der Ansprüche 1 - 4, dadurch gekennzeichnet, dass die Mikrokapseln (4) einen Durchmesser von etwa 500 - 800µ aufweisen.

6. Verfahren zur automatischen Herstellung des Wirkstoffspenders nach Anspruch 1, dadurch gekennzeichnet, dass in einer Fabrikationsstrasse zunächst eine Lage (1') saugfähigen, luftdurchlässigen Materials mittels einer Presseinrichtung (11) wenigstens eine schalenförmige Verformung (1b) erhält, welche anschliessend durch eine Fülleinrichtung (12) mit Mikrokapseln (4) beschickt und dann mittels einer ersten Kaschiereinrichtung (13) mit einer das Verschlussplättchen (2) bildenden zweiten Lage (2') überklebt wird, wonach ein Klebstoffauftragswerk (14a) die zweite Lage (2') aussenseitig mit einer Klebhaftschicht (5) versieht, die mittels einer zweiten Kaschiereinrichtung (14) mit einer abziehbaren Schutzfolie (6') überdeckt wird, und dass die schalenartige Verformung (1b) wenigstens vor Beginn ihrer Beschickung mit durch manuellen Druck zerplatzbaren Mikrokapseln (4) bis zu ihrer Ueberklebung mit der zweiten Lage (2') durch ein gegengleiches Lagerbett (20a) einer Trägerplatte (20) mit Saugluft beaufschlagt wird, die die luftdurchlässige Lage (1') im Bereich ihrer Verformung (1b) durchströmt.

7. Verfahren nach Anspruch 6, dadurch gekennzeichnet, dass die Trägerplatte (20) auf dem sich etwa von der Prägepresse (11) bis zu einer automatischen Entnahmeeinrichtung (16) erstreckenden Längsbereich der Fabrikationsstrasse verfahren wird, dass die Saugluftbeaufschlagung des Lagerbetts zum Zeitpunkt der Produktentnahme in eine Druckluftbeaufschlagung umgesteuert wird, wodurch der Wirkstoffspender aus der Trägerplatte (20) heraus gegen ein mit Saugluft beaufschlagtes Uebernameplättchen (16a) der Entnahmeeinrichtung (16) gedrückt wird.

8. Verfahren nach Anspruch 6, dadurch gekennzeichnet, dass die Beschickung der schalenartigen Verformung (1b) mit Mikrokapseln (4) mittels einer saugheberartigen, von einem Dosierbehälter (12a) zur schalenartigen Verformung (1b) hin und her verfahrbaren, einen einseitig offenen Dosierraum (12g) aufweisenden Dosierorgan (12b) geschieht, und dass der Dosierraum (12g) auf seinem Weg vom Dosierbehälter (12a) zur schalenartigen Verformung (1b) mit Saugluft beaufschlagt wird und auf dem Rückweg einen Druckluftimpuls erhält.

9. Verfahren nach Anspruch 8, dadurch gekennzeichnet, dass der Dosierraum (12g) mit nach oben gerichteter Oeffnung bei gleichzeitiger Saugluftbeaufschlagung pneumatisch mit Mikrokapseln (4) beschüttet wird und während seiner Hin- und Herbewegung je eine Drehbewegung von etwa 180^{o} ausführt.

10. Verfahren nach Anspruch 6, dadurch gekennzeichnet, dass pro Arbeitstakt eine Mehrzahl von in wenigstens einem Fertigungsblock (B) im Abstand neben- und hintereinander liegenden Wirkstoffspendern (A) gefertigt wird, wobei die Lage (1) aus saugfähigem, luftdurchlässigem Material vor dem Pressvorgang um jede zu erzeugende schalenartige Verformung (1b) herum mit Trennschnitten (1c) versehen wird, derart, dass alle von den Trennschnitten (1c) umschlossenen schalenartig zu verformenden Bereiche der genannten Lage (1') untereinander stegartig verbunden bleiben, und dass die Wirkstoffspender (A) nach der Aufbringung der silikonartigen Schutzfolie (6') durch kreisförmige Schnitte (1d) aus dem Fertigungsblock (B) ausgestanzt werden.

11. Verfahren nach Anspruch 6, dadurch gekennzeichnet, dass die Abdecklage (2') oberhalb der Verformungen (1b) mittels des Klebstoffauftragswerks (14a) mit gegenüber dem Grundriss der Wirkstoffspender (A) kleinflächigeren Klebhaftschichten (5) versehen wird, sodass sich die Stanzschnitte (1d) beim Ausstanzen der Wirkstoffspender (A) in klebhaftschichtfreien Zonen befinden, und dass die Verklebung der verformten Lage (1') mit der Abdecklage (2') mittels eines unmittelbar nach der Verklebung härtenden Klebstoffs geschieht.

12. Verfahren nach Anspruch 1, dadurch gekennzeichnet, dass die Abdecklage (2') vorgängig ihrer Verklebung mit der verformten Lage (1') mit einem in erhitztem Zustand aufgetragenen Klebstoff (hot melt) beschichtet wird, und die genannte Verklebung durch eine, durch Wärmezuführung erzielte Reaktivierung der Klebstoffschicht erfolgt.

13. Fabrikationsstrasse zur automatischen Herstellung des Wirkstoffspenders nach Anspruch 1, gekennzeichnet durch eine Prägepresse (11) zur schalenartigen Verformung einer Lage (1') saugfähigen, luftdurchlässigen Materials, der eine Fülleinrichtung (12) zur Beschickung der schalenartigen Verformung (1b) mit durch manuellen Druck zerplatzbaren Mikrokapseln (4) folgt, an die sich eine erste Kaschiereinrichtung (13) zur Ueberklebung der gefüllten Verformung (1b) mit einer Abdecklage (2') anschliesst, welcher ein Klebstoffauftragswerk (14b) zur Aufbringung einer Klebhaftschicht (5) aussenseitig auf die Abdecklage (2'), eine zweite Kaschiereinrichtung (14) zur Abdeckung der Klebhaftschicht (5) mit einer silikonartigen abziehbaren Schutzfolie (6'), sowie eine Einrichtung (16) zur automatischen Entnahme des fertigen Wirkstoffspenders (A) folgen, weiterhin durch in Materialflussrichtung (P) etwa von der Prägepresse (11) bis zur Entnahmeeinrichtung (16) verfahrbare, die schalenartige Verformung (1b) der verformten Lage (1') in gegengleichen Lagerbetten (20a) aufnehmende Trägerplatten (20), welche Lagerbetten (20a) luftdurchlässig ausgebildet und von ihrer Unterseite her mit einer Einrichtung (20b, 20c, 21, 22) zur Saugluftbeaufschlagung verbunden sind.

14. Fabrikationsstrasse nach Anspruch 13, dadurch gekennzeichnet, dass die Lagerbetten (20a) der Trägerplatten (20) aus porösen Einsätzen (20b) wie Sieb- bzw. Sintermetallplättchen bestehen.

15. Fabrikationsstrasse nach Anspruch 13, dadurch gekennzeichnet, dass die Einrichtung (20b, 20c, 21, 22) zur Saugluftbeaufschlagung der Trägerplattenlagerbetten (20a) Mittel zur Umschaltung auf Druckluft aufweist.

16. Fabrikationsstrasse nach Anspruch 13, dadurch gekennzeichnet, dass die Fülleinrichtung zur Beschickung der schalenartigen Verformung (1b) ein saugheberartiges, von einem Dosierbehälter (12a) zur schalenartigen Verformung (1b) hin und her verfahrbares, einen einseitig offenen Dosierraum (12g) besitzendes Dosierorgan (21b) aufweist, und dass der Dosierraum (12g) an ein umsteuerbares Saug-/Druckluftsystem angeschlossen ist.

17. Fabrikationsstrasse nach Anspruch 16, dadurch gekennzeichnet, dass das saugheberartige Dosierorgan um 180 schwenkbar bzw. drehbar gelagert ist und während des Dosierens im Dosierbehälter (12a) eine Position mit nach oben weisender Dosierraumöffnung einnimmt, und dass der Dosierbehälter (12a) eine Einrichtung (12h, 12i) zur pneumatischen Beschüttung des Dosierraumes (12g) mit Mikrokapseln (4) aufweist.

18. Fabrikationsstrasse nach Anspruch 16, dadurch gekennzeichnet, dass das saugheberartige Dosierorgan (12b) einen porösen Einsatz (12e) wie Sieb- bzw. Sintermetallplättchen aufweist.

19. Fabrikationsstrasse nach Anspruch 13, dadurch gekennzeichnet, dass die Kaschiereinrichtung (13) zur Ueberklebung der gefüllten Verformung (1b) mit der Abdecklage (2') ein, eine vorgängig auf die Abdecklage (2') aufgebrachte, erhärtete Klebstoffschicht reaktivierendes Pressorgan (13f) wie Heissstempel, bzw. Ultraschall- Sonotrode aufweist.

20. Fabrikationsstrasse nach Anspruch 13, dadurch gekennzeichnet, dass sie zur Herstellung einer Mehrzahl von einen zusammenhängenden Montageblock (B) bildenden, in Längs- und Querreihen zueinander liegenden Wirkstoffspendern (A) ausgelegt ist, und dass sich vor der Prägepresse (11) eine für jeden Wirkstoffspender (A) des Montageblocks (B) einen Schneidstempel aufweisende Stanzvorrichtung (10) befindet, wobei die Schneidstempel so zueinander angeordnet und ausgebildet sind, dass sie in der saugfähigen Lage (1') um jede zu erzeugende Verformung (1b) herum Trennschnitte (1c) anbringen, derart, dass alle Flächenbereiche stegartig untereinander verbunden bleiben, weiterhin durch eine vor der Entnahmeeinrichtung (16) angeordnete Stanzeinrichtung (15) zum Ausstanzen der fertigen Wirkstoffspender (A) aus dem Montageblock (B).

21. Fabrikationsstrasse nach Anspruch 13, dadurch gekennzeichnet, dass der Prägepresse (11) wenigstens ein die Lage (1') untergreifendes Huborgan (19) zugeordnet ist, das zwischen einer gegenüber der Oberfläche einer Matrizenplatte der Prägepresse (11) versenkten Position und einer die genannte Oberfläche wenigstens um das Tiefenmass der Konkavitäten der Matrizenplatte überragenden Position bewegbar gelagert ist, um die Lage (1') nach ihrer Verformung zwecks Weitertransports aus der Matrizenplatte herauszuheben.

22. Fabrikationsstrasse nach Anspruch 21, dadurch gekennzeichnet, dass das Huborgan (19) als quer zur Richtung (P) des Materialtransports ausgerichteter Stab ausgebildet und mit Ausnehmungen zum Passieren der Verformungen (1b) während des Weitertransports der verformten Lage (1') versehen ist.

23. Fabrikationsstrasse nach Anspruch 16, dadurch gekennzeichnet, dass der Dosierbehälter (12a) wenigstens zwei getrennte Kompartimente (nicht dargestellt) zur gleichzeitigen Beschickung verschiedener Dosierräume (12g) des Dosierorgans (12c) mit Mikrokapseln (4) verschiedener Wirkstoffüllungen aufweist.

## Claims

1. A dispenser for placing in the nasal region for vapourisation of active substances to be inhaled of the medical, paramedical or aromatherapeutic type, consisting of a hollow body formed as a small hollow plate (A) containing the active substance, with at least one absorbent vapourising wall, which applies the active substance to the air breathed passing along it, whereby the hollow body is provided with a flat dish (1) of air-permeable material, with a rim (1a) running around it, characterised in that the material forming the dish (1) is absorbent, that the dish (1) is covered by a closure plate (2) which is bonded thereto and together therewith encloses a chamber (3) which contains microcapsules filled with the active substance and which can be burst by manual pressure, and that the exterior of the closure plate (2) is provided with an adhesive coating (5) which adheres to the skin, and is protected by means of a foil (6) which can be pulled off.

2. Dispenser according to claim 1, characterised in that the adhesive coating (5) is dimensioned to be smaller in area than the closure plate (2), so that the entire perimeter region of the closure plate (2) is free of adhesive coating, and that the protective foil (6) and the closure plate (2) are the same in size and covering afforded.

3. Dispenser according to claim 2, characterised in that the closure plate (2) is provided with an adhesive coating (5) which is approximately in the middle, and approximately dot-like.

4. Dispenser according to one of claims 1 - 3, characterised in that the small hollow plate (A) is provided with a circular exterior contour with a diameter of about 15 - 20mm.

5. Dispenser according to one of claims 1 - 4, characterised in that the microcapsules (4) have a diameter of about 500 - 800 µ.

6. Method for the automated manufacture of the active substance dispenser according to claim 1, characterised in that on a production line firstly a layer (1') of absorbent, air-permeable material receives at least one dish-shaped moulding (1b) by means of a pressing device (11), which is subsequently loaded with microcapsules (4) by means of a filling device (12) and then, by means of a first laminating device (13) a second layer (2') forming the closure plate (2) is bonded thereover, after which an adhesive applicator (14a) provides the exterior of the second layer (2') with an adhesive coating (5), which, by means of a second laminating device (14) is covered with a pull-off protective foil (6'), and that the dish-like moulding (1b) at least before the start of its loading with microcapsules which can be burst by manual pressure (4) until its bonding over with the second layer (2'), has air suction applied to it by a counter balanced bearing bed (20a) of a carrier plate (20), which flows through the air permeable layer (1') in the area of the moulding (1b).

7. Method according to claim 6, characterised in that the longitudinal area of the production line extending approximately from the embossing press (11) to an automatic removal device (16) is operated on the carrier plate (20), and that when the product is removed, the air suction applied by the bearing bed is reversed to apply compressed air, whereby the active substance dispenser is pressed out of the carrier plate (20) against a small receiving plate (16A) to which air suction is applied, of the receiving device (16).

8. Method according to claim 6, characterised in that the loading of the dish-like moulding (1b) with microcapsules (4) is carried out by means of a suction lifting type dispensing member (12b) which is moveable to and from a dispensing container (12a) to the dish-like moulding (1b), and is provided with a dispensing chamber which is open on one side, and that the dispensing chamber (12g) is supplied with air suction on its path from the dispensing container (12a) to the dish-like moulding (1b) and on the way back receives a pulse of compressed air.

9. Method according to claim 8, characterised in that the dispensing chamber (12g) with an upwardly-facing opening is pneumatically covered with microcapsules (4) during simultaneous application of air suction and during its movement to-and-fro carries out a rotary motion of about 180°.

10. Method according to claim 6, characterised in that for each processing phase a plurality of dispensers (A) are produced, located at a distance next to and one behind the other, in at least one production block (B), whereby before the pressing process, the layer (1) of absorbent, air permeable material is provided with separating cuts (1c) around each of the dish-like mouldings (1b) to be created, such that all of the areas of the said layer (1') to be moulded, surrounded by the separating cuts (1c) remain connected to one another in a tab-wise manner, and that after receiving the silicon-type protective foil (6), the active substance dispenser (A) is punched out of the production block (B) by means of circular cuts (1d).

11. Method according to claim 6, characterised in that the covering layer (2') over the mouldings (1b) is provided, by means of the adhesive applicator (14a), with adhesive coatings having a smaller area than the surface area of the active substance dispenser (A), so that when the active substance dispenser (A) is punched out, the punch cuts (1d) are located in areas which are free of adhesive coating and that the bonding of the dish-shaped layer (1') to the covering layer (2') is by means of adhesive which hardens directly after the bonding.

12. Method according to claim 1, characterised in that during bonding thereof with the moulded layer (1'), the covering layer (2') is coated with an adhesive applied in a heated state (hot melt), and said bonding takes place by means of reactivation brought about by the application of heat to the adhesive coating.

13. Production line for automated manufacture of the active substance dispenser according to claim 1, characterised by an embossing press (11) for the dish-like moulding of a layer (1') of absorbent, air permeable material, which is followed by a filling device (12) for loading the dish-like moulding (1b) with microcapsules which can be burst by manual pressure (4), onto which is connected a first laminating device (13) for the bonding over of the filled moulding (1b) with a covering layer (2'), after which there follow an adhesive applicator (14b) for applying an adhesive layer (5) to the exterior of the covering layer (2'), a second laminating device (14) for covering the adhesive coating (5) with a silicon-type pull-off foil (6'), as well as a device (16) for automatically removing the finished active substance dispenser (A), and in addition carrier plates (20) which are moveable in the direction of the material flow (P) approximately from the embossing press (11) to the removal device (16) and incorporates the dish-like moulding (1b) of the moulded layer (1') on counter balanced bearing beds (20a), which bearing beds (20a) are formed so as to be air permeable and are connected on their underside to a device (20b, 20c, 21, 22) for applying air suction.

14. Production line according to claim 13, characterised in that the bearing beds (20a) of the carrier plates (20) consist of porous inserts (20b) such as small metal filter plates or small sintered metal plates.

15. Production line according to claim 13, characterised in that the device (20b, 20c, 21, 22) for applying air suction to the carrier plate bearing beds (20a) is provided with reversing means for switching over to compressed air.

16. Production line according to claim 13, characterised in that the filling device for loading the dish-like moulding (1b) is provided with a suction lifting type dispensing member (21b) which is moveable to and from a dispensing container (12a) to the dish-like moulding (1b) and has a dispensing chamber (12g) open on one side, and that the dispensing chamber (12g) is connected to a reversibly regulatable suction/compressed air system.

17. Production line according to claim 16, characterised in that the suction lifting type dispensing member is mounted to swivel or rotate through 180° and during dispensing takes up a position in the dispensing container (12a) with the opening of the dispensing chamber facing upwards, and that the dispensing container (12a) is provided with a device (12h, 12i) for the pneumatic covering of the dispensing chamber (12g) with microcapsules (4).

18. Production line according to claim 16, characterised in that the suction lifting type dispensing member (12b) is provided with a porous insert (12e) such a small metal filter plate or small sintered metal plate.

19. Production line according to claim 13, characterised in that the laminating device (13) for the bonding over of the filled moulding (1b) with the covering layer (2'), is provided with a pressing member (13f) such as a hot stamp or ultra sound sonotrode, which during the process is placed on the covering layer (2') and which reactivates the hardened adhesive_coating.

20. Production line according to claim 13, characterised in that it is designed for the manufacture of a plurality of active substance dispensers (A) located in longitudinal and transverse rows, forming a coherent assembly block, and that a punching device (10) provided with a cutting stamp is located in front of the embossing press (11) for each of the active substance dispensers (A) of the assembly block (B), whereby the cutting stamps are so arranged and formed in relation to one another, that they make separating cuts (1c) in the absorbent layer (1') around where each moulding (1b) is to be created, so that all the surface areas remain connected in a tab-wise manner, and furthermore by a punching device (15) which is arranged in front of the removing device (16), for punching out the finished active substance dispenser (A) from the assembly block (B).

21. Production line according to claim 13, characterised in that the embossing press (11) is assigned to at least one lifting member (19) which extends beneath the layer (1'), and which is movably mounted between a position lower with respect to the surface of a bottom die of the embossing press (11) and a position at least above the depth of the concave surfaces of the bottom die in order to lift the layer (1') after moulding thereof out of the bottom die for further transportation.

22. Production line according to claim 21, characterised in that the lifting member (19) is formed as a rod which extends at right angles to the direction (P) of the material transport, and is provided with recesses to fit the mouldings (1b) during transportation of the moulded layer (1').

23. Production line according to claim 16, characterised in that the dispensing container (12a) is provided with at least two separate compartments (not shown) for simultaneous loading of different dispensing chambers (12g) of the dispensing member (12c) with microcapsules (4) with different active substance content.

## Revendications

1. Diffuseur à placer dans la zone de l'entrée du nez pour vaporiser des principes actifs à inhaler de type médical, paramédical ou aromathérapeutique, comprenant un corps creux, réalisé sous la forme d'une plaquette creuse (A) comprenant le principe actif, avec au moins une paroi de vaporisation absorbante mélangeant l'air de respiration passant devant elle avec le principe actif, ledit corps creux présentant une cuvette (1) plate munie d'un bord circonférenciel (1a) et réalisée en un matériau perméable à l'air
**caractérisé en ce que** le matériau constituant la cuvette (1) est absorbant, en ce que la cuvette (1) est recouverte par une plaquette de fermeture (2) collée sur cette première, fermant ainsi une chambre (3) contenant le principe actif sous forme de micro-capsules (4) pouvant éclater sous compression manuelle, et en ce que la plaquette de fermeture (2) est munie d'une couche adhésive (5) adhérant sur la peau et protégée par un film (6) pouvant être arraché.

2. Diffuseur selon la revendication 1, **caractérisé en ce que** la surface de la couche adhésive (5) est relativement réduite par rapport à celle de la plaquette de fermeture (2) de manière que la zone circonférencielle totale de la plaque de fermeture (2) soit exempte de couche adhésive, et en ce que le film de protection (6) et la plaquette de fermeture (2) ont les mêmes tailles et dimensions.

3. Diffuseur selon la revendication 2, **caractérisé en ce que** la plaquette de fermeture (2) est munie d'une couche adhésive (5) de forme sensiblement ponctuelle, disposée approximativement en son milieu.

4. Diffuseur selon l'une quelconque des revendications 1 à 3, **caractérisé en ce que** la plaquette creuse (A) présente un contour extérieur circulaire ayant un diamètre d'environ 15 à 20 mm.

5. Diffuseur selon l'une quelconque des revendications 1 à 4, **caractérisé en ce que** les micro-capsules (4) présentent un diamètre d'environ 500 à 800 m.

6. Procédé de fabrication automatique du diffuseur de principes actifs selon la revendication 1, **caractérisé en ce qu'**une couche (1) de matériau absorbant, perméable à l'air, est traitée sur une chaîne de fabrication, par une presse (11), de manière à présenter au moins une déformation (1b) en forme de cuvette remplie ensuite de micro-capsules (4) au moyen d'un dispositif de remplissage (12) et recouverte ensuite, au moyen d'un premier dispositif de contrecollage (13), d'une deuxième couche (2') constituant la plaquette de fermeture (2), la deuxième couche (2') étant ensuite munie extérieurement d'une couche adhésive (5) au moyen d'un dispositif d'application d'adhésif (14a), cette couche adhésive étant recouverte, au moyen d'un deuxième dispositif de contrecollage (14), d'un film de protection (6') pouvant être arraché, et en ce que la déformation (1b) en forme de cuvette est soumise à l'effet d'un système d'air d'aspiration, au moins avant le début de son remplissage avec des micro-capsules (4) éclatant sous compression manuelle jusqu'à son recouvrement par contrecollage de la deuxième couche (2') à l'aide d'un élément d'appui opposé (20a) d'une plaque de support (20), ledit air d'aspiration traversant la couche (1') perméable à l'air dans la zone de sa déformation (1b).

7. Procédé selon la revendication 6, **caractérisé en ce que** la plaque support (20) est déplacée, dans le sens longitudinal de la chaîne de fabrication, approximativement depuis la presse (11) jusqu'à un dispositif de préhension automatique (16), en ce que l'air d'aspiration auquel est soumis l'élément d'appui est remplacé par de l'air comprimé, au moment de la préhension du produit, provoquant ainsi le soulèvement du diffuseur de principes actifs de la plaque support (20) et son appui contre une plaquette de transfert (16a) soumise à l'effet de l'air d'aspiration du dispositif de préhension (16).

8. Procédé selon la revendication 6, **caractérisé en ce que** le remplissage de la déformation (1b) en forme de cuvette avec des micro-capsules (4) s'effectue au moyen d'un organe de dosage (12b) réalisé sous la forme d'une pipette aspirante, présentant un volume de dosage (12g) ouvert sur un côté et pouvant être déplacé entre un réservoir de dosage (12a) et la déformation (1b) en forme de cuvette, et en ce que le volume de dosage (12g) est soumis à l'effet d'une aspiration sur son trajet entre le réservoir de dosage (12a) et la déformation (1b) en forme de cuvette, alors qu'il est soumis à une impulsion d'air comprimé sur son trajet de retour.

9. Procédé selon la revendication 8, **caractérisé en ce que** le volume de dosage (12g) est rempli de micro-capsules (4) par son ouverture orientée vers le haut, tout en étant soumis à l'effet de l'air d'aspiration, et en ce qu'il effectue une rotation d'environ 180° au cours de son déplacement.

10. Procédé selon la revendication 6, **caractérisé en ce que**, pour chaque cycle de travail, une pluralité de diffuseurs (A) de principes actifs sont disposés en au moins un bloc de fabrication (B) à une distance donnée entre eux, la couche (1) en matériau absorbant et perméable à l'air étant munie, avant son passage sous la presse, d'entailles de séparation (1c) autour de chaque déformation (1b) en forme de cuvette de manière que toutes les zones à déformer en cuvette entourées par les entailles de séparation (1c) de ladite couche (1') restent reliées entre elles comme par des croisillons, et en ce que les diffuseurs (A) de principes actifs sont découpés du bloc de fabrication (B) par des entailles circulaires (1d) après application du film de protection (6') fabriqué en un matériau à base de silicone.

11. Procédé selon la revendication 6, **caractérisé en ce que** la couche de recouvrement (2') est munie, au-dessus des déformations (1b), de couches adhésives (5) de surface réduite par rapport à la surface du diffuseur (A), au moyen du dispositif d'application d'adhésif (14a) de manière que les entailles de découpage (1d) se situent dans des zones exemptes de couche adhésive lors de la découpe des diffuseurs (A) de principes actifs, et en ce que le contrecollage de la couche (1') déformée sur la couche de recouvrement (2') est effectué au moyen d'une colle durcissant immédiatement après l'encollage.

12. Procédé selon la revendication 1, **caractérisé en ce que** la couche de recouvrement (2') est recouverte, avant son contrecollage sur la couche déformée (1'), d'une colle appliquée à chaud (hot melt) et en ce que ledit contrecollage est effectué par une réactivation de ladite colle sous l'effet de chaleur.

13. Chaîne de fabrication pour la fabrication automatique du diffuseur de principes actifs selon la revendication 1, **caractérisée par** une presse (11) permettant de mettre en forme de cuvette une couche (1') en un matériau absorbant et perméable à l'air, par un dispositif de remplissage (12) monté derrière ladite presse, permettant de remplir les déformations (1b) en forme de cuvette avec des micro-capsules (4) éclatant sous compression manuelle, suivi d'un premier dispositif de contrecollage (13) permettant de recouvrir la déformation (1b) remplie de micro-capsules (4) d'une couche de recouvrement (2'), suivi d'un dispositif d'application d'adhésif (14b) permettant d'appliquer une couche adhésive (5) sur la face extérieure de la couche de recouvrement (2'), suivi d'un deuxième dispositif de contrecollage (14) permettant de recouvrir la couche adhésive (5) d'un film de protection (6') pouvant être arraché, fabriqué en un matériau à base de silicone, suivi d'un dispositif de préhension automatique (16) des diffuseurs (A) de principes actifs assemblés à l'état fini, ainsi que par des plaques de support (20) se déplaçant, dans le sens d'avance de la chaîne de fabrication (P), approximativement depuis la presse (11) jusqu'au dispositif de préhension (16) et supportant les déformations (1b) en forme de cuvette de la couche déformée (1') sur des éléments d'appui opposés (20a), ces derniers étant perméables à l'air et raccordés, du côté inférieur, à un dispositif d'aspiration (20b, 20c, 21, 22).

14. Chaîne de fabrication selon la revendication 13, **caractérisée en ce que** les éléments d'appui (20a) des plaques de support (20) sont réalisés par des inserts (20b) poreux, tels que des plaquettes perforées ou des plaquettes en métal fritté.

15. Chaîne de fabrication selon la revendication 13, **caractérisée en ce que** le dispositif (20b, 20c, 21, 22) permettant de soumettre les éléments d'appui (20a) de la plaque support à de l'air d'aspiration présente des moyens pour remplacer l'air d'aspiration par de l'air comprimé.

16. Chaîne de fabrication selon la revendication 13, **caractérisée en ce que** le dispositif de remplissage des déformations (1b) en forme de cuvette comporte un organe de dosage (12b) sous forme de pipette aspirante, pouvant être déplacé entre un réservoir de dosage (12a) et la déformation (1b) en forme de cuvette et présentant un volume de dosage (12g) ouvert sur un côté, et en ce que ledit volume de dosage (12g) est raccordé à un système pouvant être commuté entre l'air d'aspiration et l'air comprimé.

17. Chaîne de fabrication selon la revendication 16, **caractérisée en ce que** l'organe de dosage sous forme de pipette aspirante peut être tourné de 180° et prendre, pendant le remplissage dans le réservoir de dosage (12a), une position dans laquelle l'ouverture du volume de dosage est orientée vers le haut, et en ce que le réservoir de dosage (12a) présente un dispositif (12h, 12i) permettant de remplir le volume de dosage (12g) de micro-capsules (4) par effet pneumatique.

18. Chaîne de fabrication selon la revendication 16, **caractérisée en ce que** l'organe de dosage (12b) du type pipette aspirante comporte un insert poreux (12e) tel que des plaquettes perforées ou des plaquettes en métal fritté.

19. Chaîne de fabrication selon la revendication 13, **caractérisée en ce que** le dispositif de contrecollage (13) permettant de recouvrir la déformation (1b) remplie de micro-capsules (4) d'une couche de recouvrement (2') présente un organe de compression (13f), tel qu'un poinçon chaud ou une sonotrode à ultra-son, permettant de réactiver une couche d'adhésif durci préalablement appliquée sur la couche de recouvrement (2').

20. Chaîne de fabrication selon la revendication 13, **caractérisée en ce qu**'elle est conçue pour fabriquer une pluralité de diffuseurs (A) de principes actifs constituant un bloc de montage (B) cohérent et disposés en rangées longitudinales et transversales, et en ce qu'un dispositif de découpage (10) est monté en face de la presse (11) et présente un poinçon de découpage pour chaque diffuseur (A) de principes actifs du bloc de montage (B), les poinçons de découpage étant disposés les uns par rapport aux autres et réalisés de manière à former des entailles de séparation (1c) dans la couche absorbante (1), autour de chaque déformation (1b) à créer, de manière que toutes les zones de la surface à déformer restent reliées entre elles par des croisillons, et en ce qu'un dispositif de découpage (15) est disposé en face du dispositif de préhension (16) pour découper les diffuseurs (A) d'agents actifs dans le bloc de montage (B) après leur assemblage complet.

21. Chaîne de fabrication selon la revendication 13, **caractérisée en ce que** la presse (11) est munie d'au moins un organe de soulèvement (19) passant au-dessous de la couche (1'), cet organe pouvant être déplacé entre une position abaissée par rapport à la surface d'une plaque de matriçage de la presse (11) et une position surélevée par rapport à ladite surface d'au moins la profondeur de la concavité de la plaque de matriçage pour soulever la couche (1'), après sa déformation, et permettre son transport ultérieur depuis la plaque de matriçage.

22. Chaîne de fabrication selon la revendication 21, **caractérisée en ce que** l'organe de soulèvement (19) est réalisé sous la forme d'une barrette orientée dans le sens transversal par rapport au sens (P) d'avance des matériaux et en ce que cette barrette est munie d'évidements pour s'adapter aux déformations (1b) pendant le transport ultérieur de la couche déformée (1').

23. Chaîne de fabrication selon la revendication 16, **caractérisée en ce que** le réservoir de dosage (12a) présente au moins deux compartiments distincts (non représentés) permettant d'approvisionner simultanément des chambres de dosage (12g) différentes de l'organe de dosage (12c) de micro-capsules (4) remplies de différents principes actifs.
